(19) Europäisches Patentamt
European Patent Office
Office européen des brevets

(11) Numéro de publication: **0 069 685**
**B1**

# (12) FASCICULE DE BREVET EUROPEEN

(45) Date de publication du fascicule du brevet: **17.09.86**

(51) Int. Cl.⁴: **B 41 M 5/26**, C 07 D 249/18

(21) Numéro de dépôt: **82420083.6**

(22) Date de dépôt: **23.06.82**

## (54) Compositions d'enregistrement thermographique les contenant et supports correspondants.

(30) Priorité: **24.06.81 FR 8112910**

(43) Date de publication de la demande: **12.01.83 Bulletin 83/2**

(45) Mention de la délivrance du brevet: **17.09.86 Bulletin 86/38**

(84) Etats contractants désignés: **BE CH DE GB IT LI**

(56) Documents cités:
**FR - A - 1 296 048**
**GB - A - 2 044 284**
**US - A - 3 413 138**

**V.GRIGNARD et al.: "Traite de chimie organique", Tome XXI, 1953, pages 737-785, Masson et Cie., Paris (FR); Chapitre: "benzotriazoles vicinaux"**
**INDUSTRIAL AND ENGINEERING CHEMISTRY, Analytical Edition, vol. 13, no. 5, Consecutive no. 40, 15 mai 1941, pages 349-351, American Chemical Society, Washington D.C. (USA); J.A.CURTIS: "Quantitative determination and separation of copper with benzotriazole"**
**CHEMICAL REVIEWS, vol. 46, 1950, The Williams & Wilkins Co., pages 1-63, Baltimore (USA); F.R.BENSON et al.: "The chemistry of the vicinal triazoles"**

(73) Titulaire: **AUSSEDAT-REY, 1 Rue du Petit Clamart, B.P.05, F-78140 Vélizy-Villacoublay (FR)**

(72) Inventeur: **Riou, Claude Raymond, 15, Chemin de Montpellaz, F-74290 Veyrier Du Lac (FR)**
Inventeur: **Fayard, Jean Florentin, 4, rue des Campanes Cran-Gevrier, F-74000 Annecy (FR)**

(74) Mandataire: **Ropital-Bonvarlet, Claude et al, Cabinet BEAU DE LOMENIE 99, Grande rue de la Guillotière, F-69007 Lyon (FR)**

(56) Documents cités: (suite)
**DERWENT JAPANESE PATENTS REPORT, Section CH., vol. 78, no. 29, 18 août 1978, J7G, page 22;**
**PATENTS ABSTRACTS OF JAPAN, vol. 4, no. 94, M20, 11 juillet 1980, page 160 M 20, Japanese Patent Office Tokyo (JP)**

**EP 0 069 685 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

Compositions d'enregistrement thermographique contenant un développeur de couleur et supports correspondants.

La présente invention concerne de nouvelles compositions d'enregistrement thermographique, ainsi que les supports d'enregistrement thermographique obtenus à partir de ces compositions. L'invention est caractérisée par l'utilisation de dérivés du benzotriazole de formule

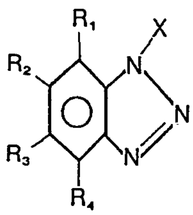

$0 \leqslant n \leqslant 4$

les substituants $R_1$, $R_2$, $R_3$, $R_4$ de la partie benzénique, qui sont identiques ou différents, pouvant être les suivants: H, alkyl, $NO_2$, halogène, acétyl, aryl, $NH_2$, OH, COOH, $SO_3H$, $NZ_1Z_2$, $COOZ_3$, $OZ_4$ ($Z_1$, $Z_2$, $Z_3$, $Z_4$ = alkyl ou aryl), X représentant H ou un groupement possédant un H labile et capable de former un anion stable par perte d'un proton $H^+$, de sels de ces composés non N-substitués ou de leurs mélanges, comme développeur de couleur dans des compositions thermoréactives comprenant un composé générateur de couleur. De préférence, X représente H, –OH, $(CH_2)_m$ –OH, avec $l \leqslant m \leqslant 10$, ou

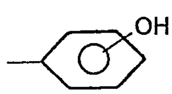

OH étant en position méta ou para l'atome d'hydrogène étant particulièrement préféré. Lors d'une élévation de température, ces dérivés de benzotriazole ou leurs sels réagissent avec le générateur de couleur pour donner une coloration irréversible. Ces deux types de produits sont conditionnés sur un support (papier ou autre) qui peut être utilisé dans tous les appareils ayant un système de visualisation thermique des informations: par exemple imprimantes de calculateurs, appareils de veille médicaux et industriels, télécopieurs...

Dans le brevet japonais 5 555 891 on a décrit un papier pour la reproduction, sensible à la chaleur, caractérisé en ce qu'il contient un 3-diéthylamino-7 (2-chloroamilino)- fluorane comme formateur de couleur et un composé triazole de formule

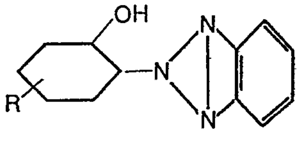

Actuellement, les utilisations industrielles du benzotriazole et de ses dérivés sont les suivantes:

– additif utilisé en faible quantité comme agent anti-corrosion (principalement du cuivre) dans les antigels et les saumures réfrigérantes; pour cette application c'est surtout le benzotriazole qui est utilisé;

– en photographie, le benzotriazole et ses dérivés sont utilisés comme «fog-inhibitor» (inhibiteur de voile); ils agissent également comme retardateur de développement; ces effets sont vraisemblablement obtenus par une réaction avec l'halogénure d'argent présent dans le film photographique;

– en photographie et également dans l'industrie des polymères et des plastiques, les dérivés du benzotriazole sont utilisés comme anti-UV. Les dérivés N-substitués du benzotriazole sont, dans ce cas, principalement utilisés, par exemple le Tinuvin 328 de formule

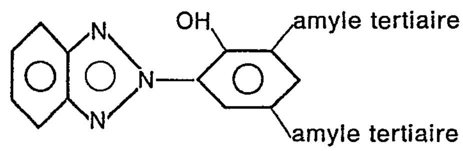

commercialisé par CIBA-GEIGY. Ces produits absorbent les radiations dans le proche ultra-violet (300 à 400 nm) et protègent ainsi de ces radiations les matériaux qui les contiennent.

Toutes ces utilisations n'ont rien à voir avec l'utilisation objet de la présente invention: développeur de générateurs de couleur dans des compositions thermoréactives.

L'augmentation récente des besoins en information et en communication a fait progresser l'utilisation et la demande de systèmes d'enregistrements, de transmission et de restitution de données. Parmi ceux-ci, les systèmes thermoréactifs connaissent un essort très important et cela pour plusieurs raisons: – le processus de formation de l'image est simple et permet d'obtenir des reproductions de bonne qualité en ce qui concerne la couleur et le contraste, – les appareils utilisés sont souvent faciles à entretenir et à utiliser, – la feuille d'enregistrement thermique, en plus de ses performances techniques remarquables, a un coût relativement faible.

Comme feuilles d'enregistrement sensibles à la chaleur pouvant être utilisées pour ces applications, on connaît celles décrites initialement dans le brevet français n° 1 440 892 (National Cash Register Company) et par la suite, dans la cascade de brevets dérivant de ce système principal, ainsi par exemple les brevets US 3 539 375 (NCR Company) et US 3 746 675 (NCR Company).

Tous ces brevets ont trait au système suivant: le chauffage induit la réaction à l'état fondu d'un leuco-colorant chromogène incolore ou de couleur pâle (générateur de couleur) et d'une substance phénolique (développeur) et conduit à la formation d'une espèce colorée.

Différents couples leuco-colorants – composés phénoliques ont été décrits pour ces applications thermographiques; pour les premiers, on peut citer: les spiropyrannes indoliniques, les lactones de colorants triphényl-méthane, des composés de la famille des fluoranes, des phtalides, les spirodi-

hydropyrannes (brevet français n° 2 272 082), les chromènes ou chromanes... Pour les seconds, on peut citer: le bisphénol A (isopropylidène -4,4′ diphénol), le p-tertiobutylphénol, le p-phénylphénol, le p,p′ (méthyl-1 -n-hexylidène) diphénol, les résines novolaques phénoliques...

On connaît également, comme compositions d'enregistrement thermo-réactif, celles contenant d'autres développeurs des leuco-colorants décrits ci-dessus, à la place des composés phénoliques, comme par exemple:

– des sels métalliques d'acides organiques et de préférence gras (résinates, acétates, phénates, stéarates, ricinoléates, oléates...). Brevet suisse n° 406 257 (NCR Company),

– des composés de structure $Z-\underset{OH}{CH}-A_1-(X_1)\ m$

Demande de brevet en FRANCE n° 7 928 857 (CIBA-GEIGY A.G.),

– des carbonates ou éthers dérivés de dihydroxy-2,3 naphtalène. Demande de brevet en FRANCE 2 427 210 (7 816 954) (LA CELLOPHANE),

– des dérivés siliciés et phosphorés du dihydroxy-2,3 naphtalène. Demande de brevet en FRANCE n° 2 427 209 (7 816 953) (LA CELLOPHANE),

– des monoaldéhydes ou polyaldéhydes substitués électro-négativement et/ou leurs produits de réaction avec un composé organique contenant des groupes hydroxyle ou bien les produits intermédiaires. Demande de brevet en FRANCE n° 2 391 858 (7 815 806) (CIBA-GEIGY A.G.).

Tous ces procédés ont bien sûr leurs qualités propres; il n'en demeure pas moins qu'ils ont pour la plupart des imperfections, notamment, en ce qui concerne la stabilité et la persistance à long terme de l'image enregistrée; en effet, la plupart de celles-ci présentent un affaiblissement plus ou moins marqué des colorations enregistrées sous l'effet de la lumière ambiante, ainsi qu'une montée du voile de fond à l'humidité et à la chaleur.

Cette stabilité de l'image enregistrée et du fond non coloré dépend bien évidemment de la nature et de la structure du générateur de couleur leuco-colorant utilisé, mais également du développeur choisi en combinaison avec lui. La combinaison retenue entre le précurseur de colorant et le révélateur a donc une grande importance sur les qualités de conservation du papier et de l'image enregistrée.

Il a été découvert selon l'invention que, dans des compositions thermoréactives, une famille tout-à-fait différente de composés, pouvait être utilisée comme développeur en association avec des précurseurs de colorants classiques.

Cette famille est constituée par les dérivés du benzotriazole de structure

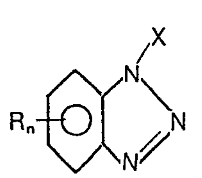

$0 \leqslant n \leqslant 4$

le ou les substituants R, identiques ou différents, pouvant être H, alkyl, aryl, $NO_2$, halogène, $NH_2$, OH, COOH, $NR_1R_2$ ($R_1$, $R_2$ = alkyl ou aryl), $COOR_3$ ($R_3$ = alkyl ou aryl), $OR_4$ ($R_4$ = alkyl ou aryl), $SO_3H$, et X représentant de préférence H, –OH, $(CH_2)_m$ –OH avec $l \leqslant m \leqslant 10$, ou

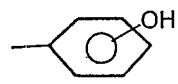

Comme exemples non limitatifs on peut citer
le benzotriazole, le méthyl-5 benzotriazole,
le méthyl-6 benzotriazole,
le phényl-5 benzotriazole,
le phényl-6 benzotriazole,
le chloro-5 benzotriazole,
le chloro-5 méthyl-6 benzotriazole,
le chloro-5 isopropyl-7 méthyl-4 benzotriazole,
le bromo-5 benzotriazole,
le nitro-4 (ou 7) benzotriazole,
le nitro-5 (ou 6) benzotriazole,
le nitro-5 diméthyl-4,7 benzotriazole,
le dinitro-4,6 (ou 5,7) benzotriazole,
l'amino-4 (ou 7) benzotriazole,
l'amino-5 (ou 6) benzotriazole,
l'amino-5 (ou 6) méthyl-6 (ou 5) benzotriazole,
l'amino-5 (ou 6) méthyl-7 (ou 4) benzotriazole,
l'amino-5 (ou 6) diméthyl-4,7 benzotriazole,
l'amino-5 chloro-4 benzotriazole,
l'amino-4 hydroxy-7 benzotriazole,
l'amino-7 carboxy-5 benzotriazole,
le diamino-4,5 (ou 6,7) benzotriazole,
l'hydroxy-4 (ou 7) benzotriazole,
l'hydroxy-5 (ou 6) benzotriazole,
le diéthoxy-4,7 benzotriazole,
l'acide dihydroxy-4,5 benzotriazolyl-7 sulfonique,
acétyl-5 benzotriazole,
acide benzotriazole-carboxylique-5 (ou 6)
et les composés analogues.

De plus amples renseignements sur ces composés, en tant que produits chimiques, peuvent être obtenus dans les publications suivantes:

– Traité de Chimie Organique de V. GRIGNARD, tome XXI, p. 737 et suivantes,

– F. BENSON et W. SAVELL, Chemical Reviews, 1950, 46, 1–68.

Ces composés dérivés du benzotriazole non N-substitués sont utilisés, selon l'invention, soit tels quels, soit sous forme de sels.

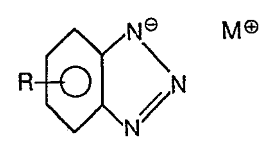

Ces sels sont obtenus par réaction directe du dérivé du benzotriazole avec un sel métallique adéquat. Les sels métalliques conduisant le plus facilement à des complexes de benzotriazole sont les sels des métaux suivants: Cu, Cd, Co, Fe (II), Ni, Mn, Zn.

Comme littérature concernant les sels métalliques de benzotriazole en tant que produits chimiques, nous pouvons citer:

– J.A. CURTIS, Ind. Eng. Chem, Anal. Ed., 1941, 13, 349,

– JE. FAGEL and G.W. EWING, J. Am. Chem. Soc. 73, 4360 (1951).

Les compositions d'enregistrement thermographique selon l'invention contiennent donc les constituants suivants:

– un ou plusieurs générateurs de couleur («color-former»):

ce produit peut appartenir à diverses familles chimiques classiquement utilisées en thermoréactif comme par exemple les lactones de colorants triphényl méthane, les fluoranes, les phtalides, les leuco-colorants de triaryl méthanes, les spiropyrannes, les chromènes, les chromanes, les leuco-colorants de phénoxazine ou phénothiazine substituée. Comme générateurs de couleur, on mentionne à titre d'exemples non limitatifs les composés suivants:

3,3-bis (p-diméthylaminophényl)-6-diméthylaminophtalide (CVL),
3,3-bis-(p-diméthylaminophényl) phtalide,
3-(p-diméthylaminophényl)-3-(1,2-diméthylindole-3-yl) phtalide,
3-(p-diméthylaminophényl)-3-(2-méthylindole-3-yl)phtalide,
3,3-bis-(1,2-diméthylindole-3-yl)-5-diméthylaminophtalide,
3,3-bis-(1,2-diméthylindole-3-yl)-6-diméthylaminophtalide,
3,3-bis-(9-éthylcarbazole-3-yl)-5-diméthylaminophtalide,
3,3-bis (2-phénylindole-3-yl)-5-diméthylaminophtalide,
3-p-diméthylaminophényl-3-(1-méthylpyrrole-2-yl)-6-diméthylaminophtalide,
éther benzylique de 4,4'-bis-diméthylaminobenzhydrine,
N-halogénophényl-leuco-auramine,
N-2,4,5-trichlorophényl-leuco-auramine,
rhodamine-B-anilinolactame,
rhodamine-(p-nitro-anilino) lactame,
rhodamine-(p-chloranilino) lactame,
7-diméthylamino-2-méthoxyfluorane,
7-diéthylamino-2-méthoxyfluorane,
7-diéthylamino-3-méthoxyfluorane,
7-diéthylamino-3-chlorofluorane,
7-diéthylamino-3-chloro-2-méthylfluorane,
7-diéthylamino-2, 3-diméthylfluorane,
7-diéthylamino-(3-acétylméthylamino)fluorane,
7-diéthylamino-(3-méthylamino)fluorane,
3,7-diéthylaminofluorane,
7-diéthylamino-3-(dibenzylamino)fluorane,
7-diéthylamino-3-(méthylbenzylamino)fluorane,
7-diéthylamino-3-(chloréthylméthyl-amino)-fluorane,
7-diéthylamino-3-(diathylamino)fluorane,
2-phénylamino-3-méthyl-6-(N-éthyl-N-p-toluyl)-amino-fluorane,
bleu de benzoyl-leucométhylène,
bleu de p-nitrobenzyl-leucométhylène,
3-méthyl-spiro-dinaphtopyrane,
3-éthyl-spiro-dinaphtopyrane,
3,3'-dichloro-spiro-dinaphtopyrane,
3-benzyl-spiro-dinaphtopyrane,
3-méthyl-naphto-(3-méthoxybenzo)-spiro-pyrane et 3-propyl-spiro-dibenzopyrane.

Les substances chromogènes incolores mentionnées ci-dessus peuvent être utilisées individuellement ou en mélange.

Les benzotriazoles utilisés selon l'invention permettent de développer d'une manière optimale tous les générateurs de couleur connus. Ce développement est optimal compte tenu du générateur de couleur utilisé. Toutefois, un certain nombre de qualités de la trace formée: couleur, stabilité, intensité ... dépendent principalement de la nature du générateur de couleur, et en particulier de la structure chimique de la famille à laquelle il appartient. Dans notre cas, le développeur de couleur de la famille du benzotriazole renforce certaines de ces qualités: intensité, stabilité à la lumière, à l'humidité, mais ne les modifie pas radicalement; ainsi par exemple, un générateur de couleur conduisant, par l'action d'un développeur de couleur classique, à un colorant peu stable à la lumière verra, par l'utilisation de dérivés du benzotriazole, sa tenue à la lumière un peu améliorée mais elle n'en deviendra pas pour autant excellente.

– un développeur de couleur, caractéristique de l'invention, appartenant à la famille des dérivés du benzotriazole:

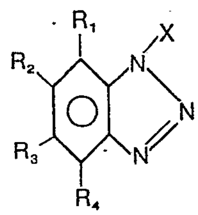

ou de leurs sels

tels que définis précédemment.

Ces compositions contiennent en outre toutes les espèces nécessaires à la formation d'une couche de bonne qualité sur le support.

– un liant polymérique dont le principal rôle est d'assurer la cohésion de l'ensemble de l'émulsion, ainsi que son accrochage sur le support; il peut intervenir également par ses caractéristiques propres sur le ramolissement de la couche. Ces liants sont soit solubles dans l'eau (le générateur de couleur et le développeur étant insolubles ou très faiblement solubles dans l'eau), dans ce cas, on peut opérer en une couche, soit solubles dans des solvants organiques, dans ce cas, il vaut mieux opérer en deux couches quand le solvant utilisé solubilise le générateur de couleur ou le développeur pour éviter toute réaction prématurée entre ces deux composés. Le générateur de couleur et le développeur sont alors placés dans des couches différentes. Ces liants peuvent être choisis dans les familles suivantes: polymères ou latex acryliques, vinyliques, cellulosiques, styréniques, halogénés, maléiques ... Comme exemples non limitatifs, nous pouvons citer: l'amidon, l'alcool polyvinylique, l'hydroxyéthyl cellulose, la méthyl cellulose, la carboxy-méthyl cellulose, la gélatine, la caséine, la gomme arabique, des sels

de copolymères de styrène et d'anhydride maléique, une émulsion d'un copolymère de styrène et de butadiène, une émulsion d'un copolymère d'acétate de vinyle et d'anhydride maléique, un copolymère de chlorure de vinylidène ...

– des cires ou composés à bas point de fusion qui servent à ajuster la température de réaction du milieu à une valeur désirée, à empêcher l'adhésion à la tête chauffante, à éviter la salissure par friction; par exemple, cires paraffiniques, cires polyoléfiniques, amides gras et leurs dérivés de méthylol, acides gras supérieurs et leurs sels métalliques, les produits de condensation d'un acide gras supérieur et d'un amine, des esters de polyalcools et d'acides gras supérieurs, des alcools supérieurs ...

– une charge pigmentaire destinée à donner une meilleure consistance à la composition de couchage, à améliorer la blancheur du fond, à réduire les problèmes de poissage de la couche et les phénomènes d'encrassement et d'usure des appareils utilisant ces émulsions; par exemple: $CaCO_3$, kaolin, talc, amidon, $TiO_2$, ZnO, $MgCO_3$, Al $(OH)_3$, argile calcinée, pigments organiques comme des polymérisats urée-formaldéhyde (par exemple «Pergopak» de CIBA-GEIGY).

– différents adjuvants couramment utilisés pour la préparation et le couchage des émulsions: dispersants, azurants, tensio-actifs, anti-mousse, plastifiants, antioxydants, anti UV, agents tamponnant le pH du milieu et stabilisant la composition comme $NaHCO_3$ ou $NH_4HCO_3$. Le choix et la nature de chacun de ces produits étant aisément effectués par un homme de l'art.

Tous ces différents constituants sont broyés, émulsionnés ou dissouts dans le milieu et couchés en une ou plusieurs couches sur un support papier ou autre (plastique par exemple). De préférence, le générateur de couleur et le développeur de couleur sont broyés séparément de manière à éviter toute réaction prématurée; les deux dispersions étant mélangées avant le couchage dans le cas d'une enduction en une couche. La nature, le grammage du support, ainsi que le nombre et l'épaisseur des couches et leur mode d'enduction dépendent de l'utilisation prévue, ainsi que de l'effet recherché, et sont aisément déterminés par un homme de l'art.

Les différents exemples donnés ci-après le sont pour illustration et ne sont en aucun cas limitatifs.

Exemple 1:

On broie séparément les compositions suivantes:

| | | | poids sec | % sec |
|---|---|---|---|---|
| 1 | Alcool polyvinylique Rhodoviol 4–20 à 5% dans l'eau (RHONE-POULENC) | 87 g | 4,4 | 25,3 |
| | Benzotriazole | 9 g | 9 | 51,7 |
| | Kaolin Ultra White 90 (ENGELHARDT) | 3,5 g | 3,5 | 20,1 |
| | Kemamide S (Stéaramide de HUMKO Chemical) | 0,4 g | 0,4 | 2,3 |
| | Anti-mousse T de BAYER | 0,1 g | 0,1 | 0,6 |
| | | | 17,4 g | 100,0 |

| | | | poids sec |
|---|---|---|---|
| 2 | Crystal Violet lactone (Reakt Violet K de BASF) | 1,6 g | 1,6 |
| | Hydroxyéthyl cellulose Cellocize QP 40 L (UNION CARBIDE) à 1% dans l'eau | 80,0 g | 0,8 |
| | Kemamide S | 1,5 g | 1,5 |
| | Lissapol N (tensioactif de ICI) | 0,1 g | 0,1 |
| | Anti-mousse T de BAYER | 0,1 g | 0,1 |
| | | | 4,1 g |

Ces deux compositions sont mélangées dans le rapport 1/1, puis couchées sur un papier de 55 g/m² à raison de 5 g/m² sec. Après séchage et éventuellement calandrage, ce papier est utilisé sur une machine à calculer de bureau à imprimante thermique OLIVETTI Logos n° 7; la trace bleue a une densité de 1,1 (mesurée à l'aide d'un densitomètre GAM RD 144 équipé d'un filtre Wratten n° 106); le seuil de réaction du papier est très marqué et se situe à 85 °C environ.

Exemple 2:

On disperse d'une manière homogène la composition suivante:

| | | | sec |
|---|---|---|---|
| Couche 1 | – Alcool polyvinylique RHODOVIOL 30–5 à 5% $H_2O$ (produit par RHONE POULENC) | 60,0 g | 3,0 g |
| | – Benzotriazole | 4,5 g | 4,5 g |
| | – Kaolin Ultra White 90 | 2,0 g | 2,0 g |
| | – Nopco 81–34 (anti-mousse de DOITTAU) | 0,1 g | 0,1 g |
| | | 66,6 g | 9,6 g |

on dépose cette couche à raison de 5 g/m² sur un support papier de 55 g/m² puis, on dépose par dessus la composition suivante, à raison de 1 g/m² sec. environ:

| | | | sec |
|---|---|---|---|
| Couche 2 | – $NH_4\ HCO_3$ | 3 g | 3 g |
| | – Color former noir PERGASCRIPT-IBR (produit de CIBA-GEIGY) | 2 g | 2 g |
| | – Kemamide S (cire amide de HUMKO Chemicals) | 3 g | 3 g |
| | – Hydroxyethylcellulose Cellocize QP 300 L à 1% dans l'eau (produit de BP Chemical) | 97 g | 0,97 g |
| | – Nopco 81–34 (anti-mousse) | 0,1 g | 0,1 g |
| | – Protesol DOS (agent d'étalement de PROTEX) | 0,1 g | 0,1 g |
| | | 105,2 g | 9,17 g |

Après séchage et éventuellement calandrage, ce papier est utilisé dans un appareil pour enregistrement d'électrocardiogrammes du type «Cardiopan 571» de PHILIPS et permet d'obtenir un tracé noir très finement résolu, quelle que soit la vitesse de défilement du papier 50, 25 ou 10 mm/s. La densité de la trace est de 1,15 (mesurée à l'aide d'un densitomètre GAM RD 144 équipé d'un filtre Wratten n° 106).

Exemple 3:

Composition identique à l'exemple 2, si ce n'est que l'on ajoute dans la couche 1:

$ZnCl_2$ 2,6 g

ce qui permet d'intensifier les traces noires obtenues thermiquement (D = 1,25) et d'en améliorer encore les caractéristiques; en particulier, nous obtenons une stabilité à la lumière remarquable; ainsi, après exposition pendant 30 heures dans un appareil de vieillissement accéléré SUNTEST 7011 de HANAU (éclairement 90 000 lux), la densité de trace est passée de 1,25 à 1,15; le fond, quant à lui, est passé de 0,08 à 0,120.

Exemple 4:

On disperse, d'une manière homogène, la composition suivante:

| | | sec | % |
|---|---|---|---|
| – Pliolite VTAC-L (copolymère vinyltoluène-acrylate vendu par GOOD YEAR) à 20% dans l'essence Exsol DSP 100–130 (ESSO CHIMIE) | 60,0 g | 12,0 g | 19,2 |
| – Benzotriazole | 18,0 g | 18,0 g | 28,9 |
| – $ZnCl_2$ | 10,4 g | 10,4 g | 16,7 |
| – Kaolin Ultra White 90 | 8,0 g | 8,0 g | 12,8 |
| – Color-former noir Pergascript IBR (CIBA-GEIGY) | 6,0 g | 6,0 g | 9,6 |
| – Kemamide S | 8,0 g | 8,0 g | 12,8 |
| – Montanox 80 (agent d'étalement de SEPPIC) | 0,1 g | 0,1 g | |
| | 110,5 g | 62,5 g | |

On broie séparément, puis on mélange avant le couchage d'une part le benzotriazole + $ZnCl_2$, et d'autre part le «color former» noir, les autres constituants étant répartis équitablement entre les deux broyages. On dépose cette couche à raison de 5 g/m² sur un support polyester de 100 µ d'épaisseur; après séchage et éventuellement calandrage, ce film thermoréactif est testé sur un appareil «THERMOTEST» de la Société SETARAM (LYON). Nous obtenons des densités de coloration de 1,20 (réflection) (Densitomètre GAM RD 144, filtre Wratten n° 106) et un seuil de réaction très marqué se situant à 85 °C environ.

Exemple 5:

On broie d'une manière homogène les compositions suivantes:

| | | |
|---|---|---|
| Couche 1 | – Ixan SGA (polychlorure de vinylidène produit par SOLVAY) | 27,3 g |
| | – Acétate d'éthyle | 110,0 g |
| | – Benzotriazole | 40,9 g |
| | – $ZnCl_2$ | 22,7 g |
| | – Kemamide S | 4,0 g |
| | – Kemamide E | 5,1 g |

On dépose cette couche à raison de 5 g/m² sec sur un support papier de 55 g/m², puis, après séchage, on dépose par dessus la composition suivante à raison de 1 g/m² sec environ:

| | | |
|---|---|---|
| Couche 2 | – Color-former Noir Pergascript IBR | 20,0 g |
| | – Alcool polyvinylique Rhodoviol 4–20 | 10,0 g |
| | – Eau | 60,0 g |
| | – Kemamide S | 30,0 g |
| | – Etingal L (anti-mousse de BASF) | 0,1 g |
| | – Protesol DO S (agent d'étalement de PROTEX) | 0,1 g |
| | – $NH_4HCO_3$ (agent stabilisant) | 30,0 g |

Après séchage et éventuellement calandrage, ce papier thermoréactif est utilisé sur un télécopieur CANON FAX 601 W; en vitesse rapide, nous obtenons des densités de noir de 0,90 et en vitesse lente des densités de 1,0. Le seuil de réactivité de ce type de papier se trouve à 75 °C environ.

Exemple 6:

On broie d'une manière homogène les compositions suivantes:

| | | |
|---|---|---|
| Couche 1 | – Alcool polyvinylique Rhodoviol 4–20 à 5% dans l'eau (produit par RHONE-POULENC) | 60,0 g |
| | – Benzotriazole | 4,5 g |
| | – $ZnCl_2$ | 2,6 g |
| | – Kaolin Ultra-White 90 | 2,0 g |
| | – Etingal L (anti-mousse de BASF) | 0,1 g |

On dépose cette couche à raison de 5 g/m² sec sur un support papier d'environ 55 g/m²; puis, on couche par dessus la composition suivante à raison de 1 g/m² sec environ:

| | | |
|---|---|---|
| Couche 2 | – Color-former Crystal Violet lactone (Reakt Violet K de BASF) | 6,0 g |
| | – Kemamide S | 5,0 g |
| | – Pliolite VTAC-L (copolymère de GOOD YEAR) à 20% dans l'essence Exsol DSP 100–130 (ESSO-CHIMIE) | 60,0 g |
| | – Montanox 80 (agent d'étalement de SEPPIC) | 0,2 g |

Après séchage et éventuellement calandrage, ce papier utilisé sur une imprimante thermique ANDERSON-JACOBSON AJ 630 (tête thermique à matrice de points 5 × 8) (10 caractères par inch et 6 lignes par inch), nous permet d'obtenir des enregistrements bleus d'une densité de 0,95 à 0,80 suivant la vitesse sélectionnée: 10, 15 ou 30 caractères par seconde (densitomètre GAM RD 144 avec filtre Wratten n° 106). Le seuil de réaction du papier est très marqué et se situe à 85 °C environ.

Exemple 7:

On disperse d'une manière homogène les compositions suivantes:

| | | | sec |
|---|---|---|---|
| Couche 1 | – Alcool polyvinylique Rhodoviol 30–5 (RHONE-POULENC) à 5% dans l'eau | 60,0 g | 3,0 g |
| | – Benzotriazole | 4,5 g | 4,5 g |
| | – Kaolin Ultra White 90 | 2,0 g | 2,0 g |
| | – Mn $(NO^3)^2$ | 3,0 g | 3,0 g |
| | – Etingal L (anti-mousse) | 0,1 g | |

On dépose cette couche à raison de 5 g/m² sec sur un support papier de 55 g/m²; puis, on couche par dessus la composition suivante à raison de 1 g/m² sec environ:

| | | |
|---|---|---|
| Couche 2 | – produit T 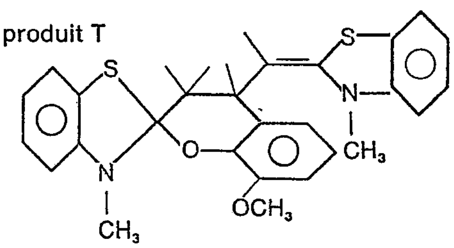 | 2,0 g |
| | – Monostéarate de glycérol | 3,0 g |
| | – Hydroxyéthylcellulose Cellocize QP 300 L à 1% dans l'eau (BP Chemical) | 97,0 g |
| | – Etingal L | 0,1 g |
| | – Protesol DOS | 0,1 g |

Après séchage et éventuellement calandrage, ce papier est utilisé dans un appareil pour enregistrements d'électrocardiogrammes du type «Cardiopan 571» de PHILIPS, et permet d'obtenir un tracé brun rouge de densité 0,80 (densitomètre GAM RD 144 avec filtre Wratten n° 106).

Exemple 8:

On disperse d'une manière homogène les compositions suivantes:

| | | |
|---|---|---|
| Couche 1 | – Ixan SGA | 27,3 g |
| | – Acétate d'éthyle | 110,0 g |
| | – Benzotriazole | 40,9 g |
| | – $CuSO_4$ | 25,0 g |
| | – Kemamide S | 9,1 g |

On dépose cette couche à raison de 5 g/m² sec environ sur un support papier de 55 g/m² environ, puis, après séchage, on dépose par dessus la composition suivante à raison de 1 g/m² sec environ:

| | | |
|---|---|---|
| Couche 2 | – Color former rouge Pergascript I6-B (CIBA-GEIGY) | 20,0 g |
| | – Alcool polyvinylique Rhodoviol 4–20 | 10,0 g |
| | – Eau | 60,0 g |
| | – Kemamide S | 30,0 g |
| | – Etingal L | 0,1 g |
| | – Protesol DOS | 0,1 g |

Après séchage et éventuellement calandrage, ce papier est utilisé sur une machine à calculer de bureau à imprimante thermique OLIVETTI Logos n° 7; la trace rouge a une densité de 1,4 (Densitomètre GAM RD 144 avec filtre Wratten vert n° 581). Le seuil de réaction de ce type de papier est bien marqué et se situe à 80 °C environ.

Les rapports des différents composés décrits dans ces exemples peuvent bien évidemment être modifiés en fonction des caractéristiques requises (sensibilité, conservation, contraste, couleur) pour le support d'enregistrement thermique, sans pour cela sortir du cadre de l'invention.

Exemple 9:

Cet exemple est identique à l'exemple 5 mais on remplace dans la couche 1 le Benzotriazole (40,9 g) par:

– Méthyl-6 Benzotriazole 45,7 g

Les résultats obtenus avec un tel papier sont quasiment identiques à ceux obtenus à l'exemple 5.

Exemple 10:

On broie d'une manière homogène les compositions suivantes:

| | | |
|---|---|---|
| Couche 1 | – Ixan SGA (Polychlorure de vinylidène produit par SOLVAY) | 27,3 g |
| | – Acétate d'éthyle | 110,0 g |
| | – Chloro-5 benzotriazole | 52,8 g |
| | – $ZnCl_2$ | 22,7 g |
| | – Kemamide S | 9,1 g |

On dépose cette couche à raison de 5 g/m² sec sur un support papier de 55 g/m² environ, puis, après séchage, on dépose par dessus la composition suivante à raison de 1 g/m² sec environ:

| | | |
|---|---|---|
| Couche 2 | – Color former Copikem XI (produit et vendu par HILTON DAVIS USA) | 20,0 g |
| | – Alcool polyvinylique Rhodoviol 4–20 | 10,0 g |
| | – Eau | 60,0 g |
| | – Kemamide S | 30,0 g |
| | – Etingal L (anti-mousse de BASF) | 0,1 g |
| | – Protesol DOS (agent d'étalement de PROTEX) | 0,1 g |
| | – $NH_4HCO_3$ | 30,0 g |

Après séchage et éventuellement calandrage, ce papier thermoréactif est testé sur un appareil THERMOTEST de la Société SETARAM (LYON); nous obtenons des densités de coloration (jaune-orangé) de 1,4 (Densitomètre GAM RD 144 avec filtre bleu Wratten n° 47); le seuil de réaction de ces papiers thermoréactifs se situe aux environs de 95 °C.

Exemple 11:

On broie de manière homogène les compositions suivantes:

| | | |
|---|---|---|
| Couche 1 | – Ixan SGA | 27,3 g |
| | – Acétate d'éthyle | 110,0 g |
| | – Diméthyl–5,6 benzotriazole–1H monohydrate | 56,7 g |
| | – $ZnCl_2$ | 22,7 g |
| | – Kemamide S | 9,1 g |

On dépose cette couche à raison de 5 g/m² sec sur un support papier de 55 g/m² environ; puis, après séchage, on dépose par dessus la composition suivante à raison de 1 g/m² sec environ:

| | | |
|---|---|---|
| Couche 2 | – Color former Olive Pergascript IG (CIBA-GEIGY) | 20,0 g |
| | – Alcool polyvinylique Rhodoviol 4–20 | 10,0 g |
| | – Eau | 60,0 g |
| | – Kemamide S | 30,0 g |
| | – Etingal L | 0,1 g |
| | – Protesol DOS | 0,1 g |
| | – $NH_4HCO_3$ | 30,0 g |

Après séchage et éventuellement calandrage, ce papier thermoréactif est testé sur un appareil THERMOTEST de la Société SETARAM (LYON); nous obtenons des densités de coloration (vert-

olive) de 1,2 (Densitomètre GAM RD 144 – filtre Wratten n° 106). Le seuil de réaction se situe aux environs de 85 °C.

Exemple 12:

On broie de manière homogène les compositions suivantes:

| | | |
|---|---|---|
| Couche 1 | – Ixan SGA | 27,3 g |
| | – Acétate d'éthyle | 110,0 g |
| | – Nitro-5 benzotriazole | 56,4 g |
| | – $ZnCl_2$ | 22,7 g |
| | – Kemamide S | 4,1 g |
| | – Kemamide E | 5,0 g |

On dépose cette couche à raison de 5 g/m² sec sur un support papier de 55 g/m² environ; puis, après séchage, on dépose par dessus la composition suivante à raison de 1 g/m² sec environ:

| | | |
|---|---|---|
| Couche 2 | – Color former Noir Pergascript IBR | 20,0 g |
| | – Alcool polyvinylique Rhodoviol 4–20 | 8,0 g |
| | – Latex Acronal 720 D (BASF) (à 50% matière sèche) | 4,0 g |
| | – Eau | 60,0 g |
| | – Kemamide S | 30,0 g |
| | – Etingal L | 0,1 g |
| | – Protesol DOS | 0,1 g |
| | – $NH_4HCO_3$ | 30,0 g |

Après séchage et éventuellement calandrage, ce papier thermoréactif est testé sur une imprimante thermique ANDERSON-JACOBSON AJ 630 (tête thermique matrice de points 5 × 8); nous obtenons des enregistrements noirs de densité de 1,10 à 0,90 suivant la vitesse sélectionnée: 10, 15 ou 30 caractères par seconde (densitomètre GAM RD 144 avec filtre Wratten n° 106). Le seuil de réaction de ces papiers se situe aux alentours de 85 °C.

En outre, pour toutes les compositions des exemples, la tenue à l'humidité est bonne; en effet, lors de stockage d'images enregistrées thermiquement à 75% d'humidité relative et 23 °C pendant trois mois, la perte de densité est inférieure à 3%.

Les propriétés des papiers exemplifiés dans les exemples ci-dessus sont rassemblées dans le tableau ci-après.

Dans ce tableau:

– DO signifie: densité mesurée du support non enregistré,

– $D_{max}$ signifie: densité mesurée de la trace.

Les tests de vieillissement accéléré ont été menés avec un appareil connu sous le nom de «SUNTEST 7011» fabriqué par la Société HANAU selon des conditions identiques comme dit pour l'exemple 3.

Tableau

| Exemple n° | Générateur de couleur | Développeur | Do | Dmax | Seuil | Couleur | Suntest hanau $T_o+30$ heures Do' | Dmax' |
|---|---|---|---|---|---|---|---|---|
| 1 | Crystal Violet lactone | Benzotriazole | 0.090 | 1.10 | 85°C | bleu | 0.090 | 0.25 |
| 2 | Noir Pergascript IBR | Benzotriazole | 0.080 | 1.15 | 95°C | noir | 0.125 | 0.915 |
| 3 | Noir Pergascript IBR | Benzotriazole $ZnCl_2$ | 0.080 | 1.25 | 95°C | noir | 0.120 | 1.15 |
| 4 | Noir Pergascript IBR | Benzotriazole $ZnCl_2$ | 0.080 | 1.20 | 85°C | noir | 0.120 | 1.10 |
| 5 | Noir Pergascript IBR | Benzotriazole $ZnCl_2$ | 0.080 | 1.20 | 75°C | noir | 0.120 | 1.10 |
| 6 | Crystal Violet lactone | Benzotriazole $ZnCl_2$ | 0.085 | 1.15 | 85°C | bleu | 0.090 | 0.45 |
| 7 | Produit T | Benzotriazole Mn $(NO_3)_2$ | 0.090 | 0.90 | 110°C | brun-rouge | 0.095 | 0.65 |
| 8 | Rouge Pergascript 16–B | Benzotriazole $CuSO_4$ | 0.090 | 1.40 (filtre Wratten 581) | 80°C | rouge | 0.120 | 1.20 |
| 9 | Noir Pergascript IBR | Methyl-6 Benzotriazole $ZnCl_2$ | 0.080 | 1.20 | 75°C | noir | 0.120 | 1.10 |

Tableau

| Exemple n° | Générateur de couleur | Développeur | Do | Dmax | Seuil | Couleur | Suntest hanau $T_o + 30$ heures Do' | Dmax' |
|---|---|---|---|---|---|---|---|---|
| 10 | Copikem XI | Chloro-5 Benzotriazole $ZnCl_2$ | 0.090 | 1.40 (filtre Wratten n° 47) | 95°C | Jaune-orangé | 0.130 | 0.65 |
| 11 | Olivo Pergascript I.G. | Dimethyl-5,6 benzotriazole –1H monohydrato $ZnCl_2$ | 0.090 | 1.20 | 85°C | vert-olive | 0.090 | 0.50 |
| 12 | Noir Pergascript IBR | Nitro-5 Benzotriazole $ZnCl_2$ | 0.080 | 1.20 | 85°C | noir | 0.120 | 1.10 |

**Revendications**

1. Compositions thermographiques contenant au moins un développeur agissant sur un générateur de couleur sous l'effet de la chaleur, caractérisées en ce que ledit développeur est

– un composé de formule:

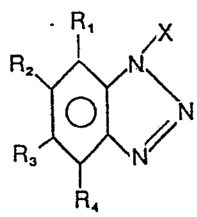

dans laquelle $R_1$, $R_2$, $R_3$, $R_4$ identiques ou différents représentent: H, alkyl, $NO_2$, halogène, acétyl, aryl, $NH_2$, OH, COOH, $SO_3H$, $NZ_1Z_2$, $COOZ_3$, $OZ_4$ ($Z_1$, $Z_2$, $Z_3$, $Z_4$ = alkyl ou aryl), X représente H ou un groupement possèdant un H labile capable de former un anion stable par perte d'un proton $H^+$

– un sel de ces composés non N-substitués

– ou leurs mélanges.

2. Compositions thermographiques, caractérisées en ce que ledit développeur selon la revendication 1 est tel que X = H, OH, $(CH_2)_m$–OH avec $1 \leqslant m \leqslant 10$ ou

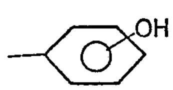

OH étant en position méta ou para.

3. Compositions thermographiques, caractérisées en ce que ledit développeur, selon les revendications 1 ou 2, est tel que X = H.

4. Compositions thermographiques, caractérisées en ce qu'elles contiennent un développeur selon l'une des revendications 1 à 3, dans lequel R est choisi parmi H, alkyl inférieur, halogène, nitro.

5. Compositions thermographiques, caractérisées en ce qu'elles contiennent un développeur selon l'une des revendications 1 à 4, dans lequel M est choisi parmi: Cu, Cd, Co, FE (II), Ni, Mn, Zn.

6. Compositions thermographiques, caractérisées en ce qu'elles contiennent un développeur selon les revendications 1 à 5 qui est choisi parmi

– le benzotriazole,
– le méthyl-6 benzotriazole,
– le chloro-5 benzotriazole,
– le diméthyl-5,6 benzotriazole – 1H monohydrate.

7. Compositions thermographiques contenant un développeur choisi parmi ceux des revendications 1 à 6 et un générateur de couleur, lesdites compositions étant caractérisées en ce que le générateur de couleur est choisi parmi les lactones de colorants triphényl méthane, les fluoranes, le phtalides, les leuco-colorants de triaryl méthanes, les spiropyrannes, les chromènes, les chromanes, les leuco-colorants de phénoxazine ou phénothiazine substituée.

8. Composition selon la revendication 7, caractérisée en ce qu'elle contient de plus un liant polymère et/ou des cires ou composés à son point de fusion, et/ou une charge pigmentaire, et/ou autres additifs classiques.

9. Composition selon l'une des revendications 7 et 8, caractérisée en ce que les couples générateur de couleur/développeur sont les suivants:

– crystal violet lactone/benzotriazole,
– générateur de couleur noir/benzotriazole,
– produit T/benzotriazole, le produit T répondant à la formule

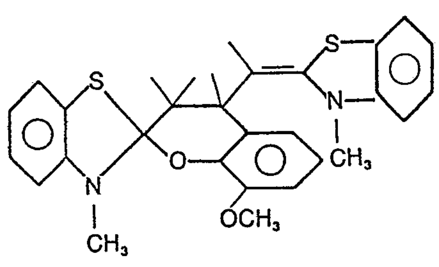

– générateur de couleur rouge/benzotriazole,
– générateur de couleur noir/méthyl-6 benzotriazole,
– générateur de couleur «COPIKEM XI»/Cl–5 benzotriazole,
– générateur de couleur olive/diméthyl-5,6 benzotriazole – 1 h monohydrate,
– générateur de couleur noir/nitro-5 benzotriazole.

10. Supports thermographiques, caractérisés en ce qu'ils contiennent une couche d'une composition selon l'une des revendications 7 à 9.

11. Supports selon la revendication 10, caractérisés en ce qu'ils contiennent, dans cet ordre:
- un support papier,
- une couche d'une composition selon l'une des revendications 7 à 9, sous la forme de deux couches séparées, la première contenant le développeur et la couche supérieure contenant le générateur.

**Claims**

1. Thermographic compositions containing at least a developer acting on a colour generator under the effect of heat, characterized in that said developer is a compound of formula:

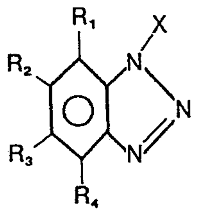

wherein $R_1$, $R_2$, $R_3$, $R_4$ identical or different represent H, alkyl, $NO_2$, halogen, acetyl, aryl, $NH_2$, COOH, $SO_3H$, $NZ_1Z_2$, $COOZ_3$, $OZ_4$ ($Z_1$, $Z_2$, $Z_3$, $Z_4$ = alkyl or aryl) X represents H or a group having a labile H capable of forming a stable anion by loss of a $H^+$ proton,
- a salt of these non N-substituted compounds
- or their mixtures.

2. Thermographic compositions, characterized in that said developer according to claim 1 is such that X = H, OH, $(CH_2)_m$–OH with $1 \leq m \leq 10$ or

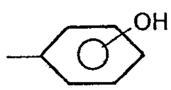

OH being in meta or para position.

3. Thermographic compositions, characterized in that said developer according to claims 1 or 2, is such that X = H.

4. Thermographic compositions, characterized in that they contain a developer according to any one of claims 1 to 3, in which R is selected from H, lower alkyl, halogen, nitro.

5. Thermographic compositions, characterized in that they contain a developer according to any one of claims 1 to 4, wherein M is selected from: Cu, Cd, Co, FE (II), Ni, Mn, Zn.

6. Thermographic compositions characterized in that they contain a developer according to claims 1 to 5, which is selected from
- benzotriazole,
- 6-methyl benzotriazole,
- 5-chloro benzotriazole,
- 5,6-dimethyl 1H-benzotriazole monohydrate.

7. Thermographic compositions containing a developer selected from among those of claims 1 to 6 and a colour generator, said compositions being characterized in that the colour generator is selected from lactones of triphenyl methane dyes, fluoranes, phthalides, leuco-dyes of triaryl methanes, spiro-pyrans, chromenes, chromanes, phenoxazine or substituted phenothiazine leuco-dyes.

8. Composition according to claim 7, characterized in that it further contains a polymer binder and/or waxes or compounds at low melting points, and/or a pigmentary filler and/or other conventional additives.

9. Composition according to any one of claims 7 and 8, characterized in that the colour-generating/developing couples are as follows:
- lactone/benzotriazole violet crystal
- black colour generator/benzotriazole
- T product/benzotriazole, the T product being of formula

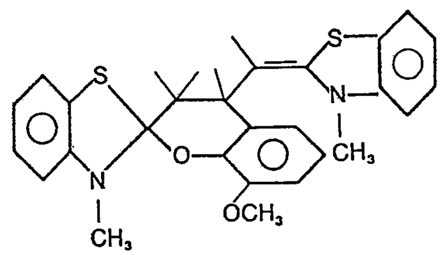

- red colour generator/benzotriazole,
- black colour generator/6-methyl benzotriazole,
- "COPIKEM XI" colour generator/Cl 5-benzotriazole,
- olive-green colour generator/5,6-dimethyl benzotriazole
- 1 h monohydrate,
- black colour generator/5-nitro benzotriazole.

10. Thermographic supports, characterized in that they contain a layer of a composition according to any one of claims 7 to 9.

11. Supports according to claim 10, characterized in that they contain, in the following order:
- a paper support,
- a layer of a composition according to any one of claims 7 to 9, in the form of two separate layers, the first layer containing the developer and the upper layer containing the generator.

**Patentansprüche**

1. Thermographische Zusammensetzungen, enthaltend mindestens einen in der Wärme auf einen Farbgenerator wirkenden Entwickler, dadurch gekennzeichnet, dass der Entwickler
- eine Verbindung der Formel:

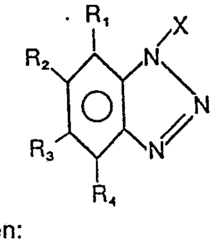

in der bedeuten:

$R_1$, $R_2$, $R_3$, $R_4$, gleich oder verschieden, H, Alkyl, $NO_2$, Halogen, Acetyl, Aryl, $NH_2$, OH, COOH, $SO_3H$,

$NZ_1Z_2$, $COOZ_3$, $OZ_4$ (wobei $Z_1$, $Z_2$, $Z_3$, $Z_4$ = Alkyl oder Aryl), X H oder eine ein labiles H aufweisende Gruppe, die durch Verlust eines Protons $H^+$ ein stabiles Anion bilden kann,

- ein Salz dieser N-nichtsubstituierten Verbindungen oder
- eines ihrer Gemische ist.

2. Thermographische Zusammensetzungen nach Anspruch 1, gekennzeichnet durch einen Entwickler, in dem X = H, OH, $(CH_2)_m$–OH und $1 \leqslant m \leqslant 10$ oder

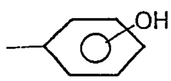

ist, wobei OH in m- oder p-Stellung ist.

3. Thermographische Zusammensetzungen nach Anspruch 1 oder 2, gekennzeichnet durch einen Entwickler, in dem X = H.

4. Thermographische Zusammensetzungen nach einem der Ansprüche 1 bis 3, gekennzeichnet durch einen Gehalt an einem Entwickler, in dem R unter H, niederem Alkyl, Halogen und Nitro auswählt wird.

5. Thermographische Zusammensetzungen nach einem der Ansprüche 1 bis 4, gekennzeichnet durch einen Gehalt an einem Entwickler, in dem M unter Cu, Cd, Co, Fe(II), Ni, Mn und Zn ausgewählt wird.

6. Thermographische Zusammensetzungen nach einem der Ansprüche 1 bis 5, gekennzeichnet durch einen Gehalt an

- Benzotriazol,
- 6-Methylbenzotriazol,
- 5-Chlorbenzotriazol,
- 5,6-Dimethylbenzotriazol-1H-monohydrat

als Entwickler.

7. Thermographische Zusammensetzungen nach einem der Ansprüche 1 bis 6, einen Farbgenerator enthaltend, dadurch gekennzeichnet, dass der Farbgenerator unter Triphenylmethanfarbstoff-Lactonen, Fluoranen, Phthaliden, Triarylmethan-Leukofarbstoffen, Spiropyranen, Chromenen, Chromanen, Phenoxazin- oder substituierten Phenothiazin-Leukofarbstoffen ausgewählt wird.

8. Zusammensetzung nach Anspruch 7, gekennzeichnet durch einen zusätzlichen Gehalt an einem polymeren Bindemittel und/oder Wachsen oder Verbindungen mit niedrigem Schmelzpunkt und/oder Pigmenten und/oder anderen üblichen Zusätzen.

9. Zusammensetzung nach Anspruch 7 oder 8, gekennzeichnet durch die folgenden Farbgenerator/Entwicklerpaare:

- Kristallviolettlacton/Benzotriazol,
- schwarzer Farbgenerator/Benzotriazol,
- Produkt T/Benzotriazol, wobei das Produkt T die folgende Formel aufweist:

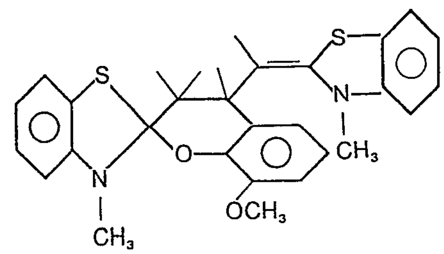

- roter Farbgenerator/Benzotriazol,
- schwarzer Farbgenerator/6-Methylbenzotriazol,
- Farbgenerator «COPIKEM XI»/Cl-5-Chlorbenzotriazol,
- olivfarbener Farbgenerator/5,6-Dimethylbenzotriazol-1H-monohydrat,
- schwarzer Farbgenerator/5-Nitrobenzotriazol.

10. Thermographischer Träger, dadurch gekennzeichnet, dass sie eine Schicht einer Zusammensetzung gemäss einem der Ansprüche 7 bis 9 enthalten.

11. Träger nach Anspruch 10, dadurch gekennzeichnet, dass sie in dieser Reihenfolge enthalten:

- ein Trägerpapier,
- eine Schicht einer Zusammensetzung gemäss einem der Ansprüche 7 bis 9, in Form zwei getrennter Schichten, wobei die erste den Entwickler und die obere Schicht den Farbgenerator enthält.